# EUROPÄISCHE PATENTSCHRIFT

(11) **EP 1 775 282 B1**
(45) Veröffentlichungstag und Bekanntmachung des Hinweises auf die Patenterteilung: **10.06.2009**
(21) Anmeldenummer: 06019726.6
(22) Anmeldetag: 21.09.2006
(51) Int. Cl.: C07C 227/18

(54) **Verfahren zur Herstellung von 4-(Aminomethyl)benzoesäuremethylester**
Process for preparing methyl 4-aminomethylbenzoate
Procédé pour préparer 4-(aminométhyl)benzoate de méthyle

(30) Priorität: 28.09.2005 DE 102005046343
(43) Veröffentlichungstag der Anmeldung: 18.04.2007
(73) Patentinhaber: Saltigo GmbH, 40764 Langenfeld (DE)
(72) Erfinder: Dockner, Michael, Dr., 50674 Köln (DE); Neugebauer, Torsten, Dr., 53604 Bad Honnef (DE)
(74) Vertreter: Pettrich, Klaus-Günter

(56) Entgegenhaltungen:
- WO-A2-98/57931

## Beschreibung

Die vorliegende Erfindung betrifft ein Verfahren zur Herstellung von 4-(Aminomethyl)benzoesäuremethylester durch Veresterung von 4-(Aminomethyl)benzoesäure und nachfolgende Gewinnung des 4-(Aminomethyl)benzoesäuremethylesters unter spezieller pH- und Temperaturkontrolle.

Die Ester der 4-(Aminomethyl)benzoesäure sind Zwischenprodukte für die Synthese von pharmazeutischen Wirkstoffen. Da Methylester leicht wieder verseift werden können, ist der 4-(Aminomethyl)benzoesäuremethylester dann von Interesse, wenn in einer Synthesesequenz zum Wirkstoff milde Bedingungen zur Freisetzung der Säurefunktion gesucht werden.

Für die Herstellung des 4-(Aminomethyl)benzoesäuremethylesters kennt die Literatur bereits verschiedene Wege.

Zugänglich ist der 4-(Aminomethyl)benzoesäuremethylester über die katalytische Hydrierung des 4-Cyanobenzoesäuremethylesters (Synlett 10, 1623 (2001)), des Oxims (JP 1979-85926, JP 1973-136140), das aus 4-Formylbenzoesäuremethylester und Hydroxylamin gewonnen wird, oder des Imins aus 4-Formylbenzoesäuremethylester und Ammoniak (JP 1977-104929).

Neben Wasserstoff wird auch Natriumborhydrid in Verbindung mit Cobalt-(II)-chlorid (J. Am. Chem. Soc. 104, 6801 (1982)) oder Trifluoressigsäure (Tetrahedron Lett. 33, 2875 (1976)) zur Reduktion der in Position 4 ständigen Nitrilgruppe bzw. Amidogruppe des Benzoesäuremethylesters verwendet.

Aus SU 1989-4670483 ist es ferner bekannt, dass die para-ständige Amidogruppe auch elektrochemisch zur Aminomethylengruppe reduziert werden kann.

In Chem. Lett. 10, 1733 (1984) wird 4-Methoxycarbonyl-benzylazid als Ausgangsverbindung beschrieben, aus der man durch Behandlung mit Natriumhydrogentellurid den 4-(Aminomethyl)benzoesäuremethylester erhält.

Durch einen Chlor-/Aminaustausch an dem para-Chlormethylbenzoesäuremethylester in nicht wässrigen Medien kann der 4-(Aminomethyl)benzoesäuremethylester ebenfalls synthetisiert werden (JP 1972-75050).

Bei allen vorgenannten Methoden ist die gewünschte Methylestergruppe in der jeweiligen Ausgangsverbindung bereits vorhanden. Aufgrund der zum Teil harschen Reaktionsbedingungen wird jedoch der Methylester unerwünschterweise bereits während der Reaktion oder der Aufarbeitung wieder verseift.

Möglich ist es auch, für die Synthese der 4-(Aminomethyl)benzoesäureester von der in kommerziellen Mengen verfügbaren 4-(Aminomethyl)benzoesäure auszugehen und diese mit dem entsprechenden Alkohol zu verestern.

Aus J. Med. Chem. 37, S.1810-1822 (O'Brien et al), (1994) ist die Veresterung in Gegenwart von Salzsäure in am Rückfluß siedendem Methanol bekannt. Das Produkt fällt allerdings als Hydrochlorid an und dieses schränkt wegen seiner korrosiven Eigenschaften die Apparateauswahl zur Isolierung des Feststoffs stark ein.

In US-A-6,172,084 wird statt Salzsäure Chlorwasserstoff in die methanolische Reaktionsmischung eingeleitet und über Nacht die 4-(Aminomethyl)benzoesäure durch Erhitzen am Rückfluß verestert. Die Reaktionsmischung wird anschließend aufkonzentriert, mit gesättigter wässriger Natriumcarbonatlösung versetzt und dreimal mit Methylenchlorid extrahiert. Die vereinigten organischen Phasen werden mit Magnesiumsulfat getrocknet, vom Trockenmittel durch Filtration befreit und anschließend aufkonzentriert. Eine Durchführung nach dieser Methode im technischen Maßstab ist nicht üblich, da große Mengen an Magnesiumsulfat anfallen, deren Entsorgung teuer ist. Ferner liegt auch die gemäß US-A-6,172,084 erzielte Ausbeute nur bei 57%.

Die beiden letztgenannten Methoden zur Herstellung von 4-(Aminomethyl)benzoesäuremethylester sind für eine Durchführung im technischen Maßstab hinsichtlich Apparateauswahl und ökologischer Gesichtspunkte nicht befriedigend. Weiterhin ist die direkte Verwendung des gemäß J. Med. Chem. 37, S.1810-1822 (O'Brien et al.) (1994) anfallenden Hydrochlorids in einer Folgestufe nicht immer möglich und macht in solchen Fällen die vorherige Freisetzung in einem weiteren separaten Schritt notwendig, wodurch die wirtschaftliche Attraktivität des Gesamtverfahrens leidet. Es bestand daher ein Bedürfnis nach einem einfach durchführbaren, wirtschaftlichen und ökologisch unkritischen Verfahren zur Herstellung von 4-(Aminomethyl)benzoesäureestern, das für eine breite Anwendung zur Verfügung steht.

Überraschenderweise wurde gefunden, dass die Herstellung von 4-(Aminomethyl)benzoesäuremethylester, die ausgehend von 4-(Aminomethyl)benzoesäure durch Veresterung in Gegenwart von Salzsäure erfolgt, auch ohne eine Zwischenisolierung des Hydrochlorids möglich ist, sofern man die Aufarbeitung des Reaktionsgemisches und die Gewinnung des 4-(Aminomethyl)benzoesäuremethylesters unter speziellen Verfahrensbedingungen durchführt.

Gegenstand der Erfindung ist somit ein Verfahren zur Herstellung von 4-(Aminomethyl)benzoesäuremethylester durch Veresterung von 4-Aminomethylbenzoesäure mit Methanol in Gegenwart von Salzsäure, wobei dieses Verfahren dadurch gekennzeichnet ist, dass man das nach der Umsetzung mit Methanol resultierende Reaktionsgemisch
1) zunächst auf eine Temperatur im Bereich von -15 bis + 10 °C und durch Zugabe einer Base auf einen pH-Wert im Bereich von 4 bis 9 einstellt,
2) es anschließend aufkonzentriert und ein organisches Lösungsmittel zugibt,
3) danach den pH-Wert der wässrigen Phase durch weitere Zugabe einer Base auf einen Wert im Bereich von 9 bis 12 einstellt, und
4) die organische Phase, welche den 4-(Aminomethyl)benzoesäuremethylester der allgemeinen Formel (1) enthält, abtrennt.

Für die erfolgreiche Durchführung des erfindungsgemäßen Verfahrens ist es essentiell, dass man das nach der Umsetzung der 4-(Aminomethyl)benzoesäure mit Methanol resultierende Reaktionsgemisch auf eine Temperatur im Bereich von -15 bis +10 °C, bevorzugt im Bereich von +5 bis +10 °C, abkühlt.

Es ist ferner essentiell, dass das Reaktionsgemisch bei dieser Temperatur im Bereich von -15 bis +10 °C, bevorzugt +5 bis +10 °C, auf einen pH-Wert im Bereich von 4 bis 9 eingestellt wird. Dies erfolgt üblicherweise durch Zugabe einer wasserlöslichen Base. Bevorzugt wird das Gemisch auf einen pH-Wert im Bereich von 5 bis 8 eingestellt, besonders bevorzugt von 6,0 bis 7,0.

Als wasserlösliche Basen kommen für die Einstellung des pH-Wertes prinzipiell wasserlösliche tertiäre Amine und Alkali- und Erdalkalioxide, -hydroxide, -carbonate, -hydrogenphosphate und - phosphate in Frage. Bevorzugt verwendet man Natrium- oder Kaliumhydroxid.

Die Konzentration der Base ist beliebig variierbar und kann beispielsweise 2 bis 50 Gew.-% betragen. Bevorzugt sind wässrige 4 bis 6 Gew.-%ige Lösungen von Kalium- oder Natriumhydroxid. Tertiäre Amine kann man auch als solche, d. h. ohne Wasser, einsetzen.

Nach der pH-Wert Einstellung wird die Reaktionsmischung unter vermindertem Druck durch Destillation aufkonzentriert. Der Druck ist dabei vorzugsweise so zu wählen, dass die Innentemperatur im Reaktionsgemisch 40 °C nicht überschreitet. Beispielsweise kann zu Beginn der Aufkonzentierung ein Druck von 200 mbar gewählt und im weiteren Verlauf auf 40 mbar erniedrigt werden. Die Innentemperatur liegt unter diesen Bedingungen im Bereich von 17 bis 33 °C.

Anschließend wird zum verbleibenden Reaktionsgemisch, dem Konzentrat, ein organisches Lösungsmittel gegeben.

Als organische Lösungsmittel können polare, mit Wasser nicht mischbare Lösungsmittel eingesetzt werden. Geeignet sind beispielsweise aromatische Kohlenwasserstoffe wie Toluol sowie halogenierte gesättigte Kohlenwasserstoffe wie Methylenchlorid.

Bei Verwendung von Toluol oder anderen aromatischen Kohlenwasserstoffen ist es sinnvoll, die wässrige Phase vorzugsweise mit Natriumchlorid oder anderen Salzen zu sättigen, um den 4-(Aminomethyl)-benzoesäuremethylester mit hoher Ausbeute in die organische Phase zu extrahieren.

Bevorzugt verwendet man als organische Lösungsmittel Methylenchlorid.

Nach der Zugabe des Lösungsmittels ist es wichtig, den pH-Wert der wässrigen Phase des resultierenden zweiphasigen Gemischs auf einen Wert im Bereich von 9 bis 12 einzustellen. Dies erfolgt üblicherweise durch Zugabe einer wasserlöslichen Base. Bevorzugt wird das Gemisch auf einen pH-Wert im Bereich von 9 bis 11, besonders bevorzugt im Bereich von 10,0 bis 11,0, eingestellt.

Als wasserlösliche Basen kommen prinzipiell wasserlösliche Alkali- und Erdalkalioxide, - hydroxide, -carbonate, -hydrogenphosphate und -phosphate in Frage. Bevorzugt verwendet man Natrium- oder Kaliumhydroxid. Von Vorteil ist es, die gleiche Base zu verwenden wie bei der Einstellung des pH-Wertes in Schritt 1) des erfindungsgemäßen Verfahrens.

Die Konzentration der Base ist beliebig variierbar und kann beispielsweise 2 bis 50 Gew.-% betragen. Bevorzugt sind wässrige 4 bis 6 Gew.-%ige Lösungen von Kalium- oder Natriumhydroxid.

Die wasserlösliche Base wird beispielsweise bei einer Temperatur des Reaktionsgemisches im Bereich von -15 bis +10 °C, bevorzugt von +5 bis +10 °C, zugegeben.

Nach der vorgenannten pH-Einstellung wird die organische Phase, welche das gewünschte Produkt in Form des gegebenenfalls substituierten 4-(Anünomethyl)-benzoesäureesters der allgemeinen Formel (1) enthält, abgetrennt. Eine zweite Extraktion mit dem zuvor verwendeten Lösungsmittel kann, muss aber nicht erfolgen.

Vor der Abtrennung der organischen Phase kann die Sättigung der wässrigen Phase mit Natriumchlorid oder anderen Salzen helfen, den 4-(Aminomethyl)benzoesäureester der Formel (1) effizienter in die organische Phase zu überführen. Das ist insbesondere dann hilfreich, wenn als organisches Lösungsmittel Toluol oder andere aromatische Kohlenwasserstoffe verwendet werden.

Durch die spezielle Verfahrensführung während der Aufarbeitung des Reaktionsgemisches nach der Veresterung gelingt es im erfindungsgemäßen Verfahren, die unerwünschte und verfrühte Verseifung des 4-(Aminomethyl)benzoesäuremethylesters zu unterdrücken und unter den gewählten Extraktionsbedingungen ferner eine optimale Überführung des gewünschten Produktes in die organische Phase sicherzustellen. Der 4-(Aminomethyl)benzoesäuremethylester kann daher im erfindungsgemäßen Verfahren in exzellenten Ausbeuten von über 85 %, bezogen auf die eingesetzte 4-(Aminomethyl)benzoesäure, erhalten werden. Bevorzugt liegen die erzielten Ausbeuten bei 88 % oder darüber.

Im erfindungsgemäßen Verfahren ist im Gegensatz zu dem aus US-A-6,178,084 bekannten Verfahren eine Trocknung der organischen Phase im Anschluß an Schritt 4) vorteilhafterweise nicht erforderlich. Der Anfall aufwändig zu entsorgender Mengen an Trocknungsmittel kann somit vermieden werden.

Das erfindungsgemäße Verfahren liefert den in einem organischen Lösungmittel gelösten, 4-(Aminomethyl)benzoesäuremethylester der allgemeinen Formel (1) ohne Zwischenisolierung des intermediär gebildeten Hydrochlorids und vermeidet auf diese Weise auch eine Einschränkung der Apparatewahl.

Ferner ist der 4-(Aminomethyl)-benzoesäuremethylester breiter einsetzbar als das entsprechende Hydrochlorid.

Es ist möglich, die organische Lösung des 4-(Aminomethyl)benzoesäuremethylesters direkt in eine sich anschließende Folgestufe einzusetzen. Es ist aber auch möglich, zuvor einen Lösungsmitteltausch vorzunehmen. Wenn es sich bei dem Lösungsmittel der organischen Lösung um Methylenchlorid handelt, ist beispielsweise ein Wechsel zu höher siedenden Lösungsmittlen wie Toluol problemlos möglich.

Sofern zeitlich keine direkte Weiterverarbeitung erfolgt, hat es sich bewährt, diese organische Lösung bei 0 bis 5 °C zu lagern, um eine Oligomerisierung zu unterdrücken.

Das erfindungsgemäße Verfahren läßt sich vorteilhaft nutzen, um den erhaltenen 4-(Aminomethyl)benzoesäuremethylester mit Cyclohexenylchlorbenzol in einer Buchwald-C-N-Kupplung umzusetzen. Gemäß US-A-6,172,084 läßt sich der 4-(Aminomethyl)benzoesäuremethylester ferner vorteilhaft für die Entwicklung von Antibiotika auf Chinolin-Indol-Basis verwenden.

### Beispiel

In einem 1200 1 Rührwerksbehälter aus Stahl-email werden 60 kg 4-(Aminomethyl)-benzoesäure, 480 kg Methanol und 89 kg 30 %ige Salzsäure am Rückfluß zum Sieden erhitzt. Nach 7 h Reaktionszeit wird das Reaktionsgemisch auf 10 °C abgekühlt.

Durch Zugabe von 290 kg 4 %iger Natronlauge wird der pH-Wert der Reaktionsmischung zunächst auf einen pH-Wert von 6-7 eingestellt, bevor unter vermindertem Druck eine Methanol/Wasser Mischung abdestilliert wird.

Anschließend werden 400 kg Methylenchlorid zugegeben und der pH-Wert der wässrigen Phase bei einer Temperatur von 5-10 °C auf einen Wert von 10-11 eingestellt.

Die untere organische Phase wird abgetrennt. Die wässrige Phase wird noch einmal mit 265 kg Methylenchlorid extrahiert.

Die organischen Phasen werden vereinigt und bei 0-5 °C gelagert.

Der Gehalt der Lösung wird mittels quantitativer HPLC Chromatographie bestimmt.
Die Ausbeute an 4-(Aminomethyl)benzoesäuremethylester beträgt 88-89 %.

## Patentansprüche

1. Verfahren zur Herstellung von 4-(Aminomethyl)benzoesäuremethylester durch Veresterung von 4-Aminomethylbenzoesäure mit Methanol in Gegenwart von Salzsäure, **dadurch gekennzeichnet, dass** man das nach der Umsetzung mit Methanol resultierende Reaktionsgemisch
1) zunächst auf eine Temperatur im Bereich von -15 bis + 10 °C und durch Zugabe einer Base auf einen pH-Wert im Bereich von 4 bis 9 einstellt,
2) es anschließend aufkonzentriert und ein organisches Lösungsmittel zugibt,
3) danach den pH-Wert der wässrigen Phase durch weitere Zugabe einer Base auf einen Wert im Bereich von 9 bis 12 einstellt und
4) die organische Phase, welche den 4-(Aminomethyl)benzoesäuremethylester enthält, abtrennt.

2. Verfahren nach Anspruch 1, **dadurch gekennzeichnet, dass** man das Reaktionsgemisch in Schritt 1) auf eine Temperatur im Bereich von 5 bis +10 °C und einen pH-Wert im Bereich von 5 bis 8, bevorzugt von 6,0 bis 7,0 einstellt.

3. Verfahren nach Anspruch 1 oder 2, **dadurch gekennzeichnet, dass** man die Einstellung des pH-Wertes in Schritt 1) durch Zugabe einer wasserlöslichen Base, bevorzugt ein wasserlösliches tertiäres Amin oder Alkali- und Erdalkalioxide, -hydroxide, -carbonate, - hydrogenphosphate und -phosphate, besonders bevorzugt Natrium- oder Kaliumhydroxid durchführt.

4. Verfahren nach einem oder mehreren der Ansprüche 1 bis 3, **dadurch gekennzeichnet, dass** man in Schritt 2) als organisches Lösungsmittel ein polares, mit Wasser nicht mischbares Lösungsmittel einsetzt, bevorzugt aromatische Kohlenwasserstoffe, besonders bevorzugt Toluol, oder halogenierte gesättigte Kohlenwasserstoffe, besonders bevorzugt Methylenchlorid.

5. Verfahren nach einem oder mehreren der Ansprüche 1 bis 4, **dadurch gekennzeichnet, dass** man in Schritt 3) den pH-Wert der wässrigen Phase des resultierenden zweiphasigen Gemischs auf einen Wert im Bereich von 9 bis 11, bevorzugt von 10,0 bis 11,0, einstellt.

6. Verfahren nach einem oder mehreren der Ansprüche 1 bis 5, **dadurch gekennzeichnet, dass** man in Schritt 3) als wasserlösliche Base wasserlösliche Alkali- oder Erdalkalioxide, - hydroxide, -carbonate, -hydrogenphosphate oder -phosphate, bevorzugt Natrium- oder Kaliumhydroxid, einsetzt.

7. Verfahren nach einem oder mehreren der Ansprüche 1 bis 6, **dadurch gekennzeichnet, dass** keine Trocknung der nach Schritt 4) abgetrennten organischen Phase, welche den 4-(Aminomethyl)benzoesäuremethylester enthält, erfolgt.

8. Verfahren nach einem oder mehreren der Ansprüche 1 bis 7, **dadurch gekennzeichnet, dass** die organische Phase aus Schritt 4), die den 4-(Aminomethyl)benzoesäuremethylester enthält, entweder nach einem Lösungsmittelaustausch oder direkt in eine sich anschließende chemische Umsetzung als Edukt eingesetzt wird.

## Claims

1. Process for preparing methyl 4-(aminomethyl)benzoate by esterifying 4-aminomethylbenzoic acid with methanol in the presence of hydrochloric acid, **characterized in that** the reaction mixture resulting after the reaction with methanol
1) is first adjusted to a temperature in the range from -15 to +10°C and, by adding a base, to a pH in the range from 4 to 9,
2) is subsequently concentrated and an organic solvent is added,
3) then the pH of the aqueous phase is adjusted to a value in the range from 9 to 12 by further addition of a base and
4) the organic phase which comprises the methyl 4-(aminomethyl)benzoate is removed.

2. Process according to Claim 1, **characterized in that** the reaction mixture in step 1) is adjusted to a temperature in the range from 5 to +10°C and a pH in the range from 5 to 8, preferably from 6.0 to 7.0.

3. Process according to Claim 1 or 2, **characterized in that** the adjustment of the pH in step 1) is carried out by adding a water-soluble base, preferably a water-soluble tertiary amine, or alkali metal and alkaline earth metal oxides, alkali metal and alkaline earth metal hydroxides, alkali metal and alkaline earth metal carbonates, alkali metal and alkaline earth metal hydrogen phosphates, and alkali metal and alkaline earth metal phosphates, more preferably sodium hydroxide or potassium hydroxide.

4. Process according to one or more of Claims 1 to 3, **characterized in that** the organic solvent used in step 2) is a polar, water-immiscible solvent, preferably aromatic hydrocarbons, more preferably toluene, or halogenated saturated hydrocarbons, more preferably methylene chloride.

5. Process according to one or more of Claims 1 to 4, **characterized in that** the pH of the aqueous phase of the resulting biphasic mixture is adjusted in step 3) to a value in the range from 9 to 11, preferably from 10.0 to 11.0.

6. Process according to one or more of Claims 1 to 5, **characterized in that** the water-soluble base used in step 3) comprises water-soluble alkali metal or alkaline earth metal oxides, alkali metal or alkaline earth metal hydroxides, alkali metal or alkaline earth metal carbonates, alkali metal or alkaline earth metal hydrogen phosphates, or alkali metal or alkaline earth metal phosphates, preferably sodium hydroxide or potassium hydroxide.

7. Process according to one or more of Claims 1 to 6, **characterized in that** the organic phase which comprises the methyl 4-(aminomethyl)benzoate and is removed after step 4) is not dried.

8. Process according to one or more of Claims 1 to 7, **characterized in that** the organic phase from step 4) which comprises the methyl 4-(aminomethyl)benzoate is used as a reactant either after a solvent exchange or directly in a subsequent chemical reaction.

## Revendications

1. Procédé pour la préparation d'ester méthylique de l'acide 4-(aminométhyl)benzoïque par estérification d'acide 4-aminométhylbenzoïque avec du méthanol en présence d'acide chlorhydrique, **caractérisé en ce que** le mélange réactionnel obtenu, après la transformation avec le méthanol
1) est d'abord réglé à une température dans la plage de -15 à +10 °C et par addition d'une base à un pH dans la plage de 4 à 9,
2) est ensuite concentré, puis on ajoute un solvant organique,
3) on règle ensuite le pH de la phase aqueuse par une nouvelle addition d'une base à une valeur dans la plage de 9 à 12 et
4) on sépare la phase organique qui contient l'ester méthylique de l'acide 4-(aminométhyl)benzoïque.

2. Procédé selon la revendication 1, **caractérisé en ce qu'**on règle le mélange réactionnel dans l'étape 1) à une température dans la plage de 5 à +10°C et à un pH dans la plage de 5 à 8, de préférence de 6,0 à 7,0.

3. Procédé selon la revendication 1 ou 2, **caractérisé en ce qu'**on réalise le réglage du pH dans l'étape 1) par addition d'une base soluble dans l'eau, de préférence une amine tertiaire soluble dans l'eau ou des oxydes, hydroxydes, carbonates, hydrogénophosphates et phosphates de métal alcalin ou alcalino-terreux, de manière particulièrement préférée l'hydroxyde de sodium ou de potassium.

4. Procédé selon l'une ou plusieurs des revendications 1 à 3, **caractérisé en ce qu'**on utilise dans l'étape 2), comme solvant organique, un solvant polaire, non miscible avec l'eau, de préférence des hydrocarbures aromatiques, de manière particulièrement préférée le toluène ou des hydrocarbures saturés halogénés, de manière particulièrement préférée le chlorure de méthylène.

5. Procédé selon l'une ou plusieurs des revendications 1 à 4, **caractérisé en ce qu'**on règle dans l'étape 3) le pH de la phase aqueuse du mélange à deux phases obtenu à une valeur dans la plage de 9 à 11, de préférence de 10,0 à 11,0.

6. Procédé selon l'une ou plusieurs des revendications 1 à 5, **caractérisé en ce qu'**on utilise dans l'étape 3) comme base soluble dans l'eau des oxydes, hydroxydes, carbonates, hydrogénophosphates ou phosphates de métal alcalin ou alcalino-terreux solubles dans l'eau, de préférence l'hydroxyde de sodium ou de potassium.

7. Procédé selon l'une ou plusieurs des revendications 1 à 6, **caractérisé en ce qu'**on ne réalise pas de séchage de la phase organique séparée après la phase 4), qui contient l'ester méthylique de l'acide 4-(aminométhyl)benzoïque.

8. Procédé selon l'une ou plusieurs des revendications 1 à 7, **caractérisé en ce que** la phase organique de l'étape 4), qui contient l'ester méthylique de l'acide 4-(aminométhyl)benzoïque, est utilisée, soit après un remplacement de solvant ou directement, comme produit de départ dans une transformation chimique consécutive.
